# EUROPÄISCHE PATENTSCHRIFT

(11) **EP 0 463 128 B1**
(45) Veröffentlichungstag und Bekanntmachung des Hinweises auf die Patenterteilung: **21.09.1994**
(21) Anmeldenummer: 91900713.8
(22) Anmeldetag: 07.01.1991
(51) Int. Cl.: G01N 21/89, G01N 33/36

(54) **VORRICHTUNG ZUR PHOTOELEKTRISCHEN ÜBERWACHUNG EINES LAUFENDEN FADENS**
DEVICE FOR PHOTO-ELECTRICALLY MONITORING A MOVING YARN
DISPOSITIF POUR LE CONTROLE PHOTOELECTRIQUE D'UN FIL DEFILANT

(30) Priorität: 12.01.1990 CH 102/90
(43) Veröffentlichungstag der Anmeldung: 02.01.1992
(73) Patentinhaber: LÖPFE AG, CH-8623 Wetzikon (CH)
(72) Erfinder: HAGMANN, Karl, CH-6020 Emmenbrücke (CH)
(74) Vertreter: Herrmann, Peter Johannes
(86) Internationale Anmeldenummer: CH9100003
(87) Internationale Veröffentlichungsnummer: WO9110898

(56) Entgegenhaltungen:
- EP-A- 0 213 587
- EP-A- 0 322 470
- EP-A- 0 322 471

## Beschreibung

Die Erfindung betrifft eine Vorrichtung zur photoelektrischen Überwachung eines laufenden Fadens auf Unregelmässigkeiten, bestehend aus in Fadenlaufrichtung hintereinander angeordneten Messköpfen, wobei dem ersten Messkopf ein Strahlensender und ein Strahlenempfänger und dem zweiten Messkopf ein Strahlensender und ein Strahlenempfänger zugeordnet sind. Siehe zB. EP-A- 0 213 587.

Es ist bekannt, dass verschiedene Unregelmässigkeiten an einem Einzelfaden verschiedene Auswirkungen bei der Weiterverarbeitung oder im Endprodukt zur Folge haben können. Flusen beispielsweise führen hauptsächlich zu Störungen bei der Weiterverarbeitung. Eine bestimmte geringe Anzahl von z.B. abstehenden Fibrillen können in bestimmten Fällen für bestimmte Verwendungszwecke von Fäden toleriert werden. Zur Qualitätsbeurteilung wäre es daher wünschenswert die einzelnen Fehler individuell zu erfassen.

Die photoelektronische Überwachung eines laufenden Fadens ist bekannt (US-A-3,729,635). Darin wird auch ein Schatten der Fadenschar in ein elektrisches Signal umgewandelt, welche die unter einer Sollgrenze liegenden Ausschläge unterdrückt und die über einer Sollgrenze liegenden Ausschläge zur Abstellen einer Textilmaschine verwendet. Die Entwicklung verlief in einer ständigen Erhöhung der Empfindlichkeit. Kleine Garnfehler können wegen des Grundrauschens auch mit erhöhter Empfindlichkeit nicht erfasst werden. Ein Faden besteht meistens aus Einzelfibrillen und weist oberflächenbedingt nie einen absolut homogenen, runden Querschnitt auf. Verdrehbewegungen, unruhiger Fadenlauf und ein nicht absolut eben liegender Faden bewirken deshalb bei der Abschattung ein breiteres, diffuseres Schattenbild als der effektive Fadenquerschnitt aufweist. Dieser Diffusanteil des Schattenbildes wird als Grundrauschen des laufenden Fadens bezeichnet. Kleine Fehler tauchen in diesem Graundrauschen unter. Dieser Grundrauschanteil bewegt sich wertmässig in der Praxis zwischen 10 und 120 % des Fadenquerschnittes.

Der Druckschrift sind keine Angaben über die Führung eines Fadens und dessen Messung auf Unregelmässigkeiten entnehmbar. Mit allen bekannten Vorrichtungen und Verfahren lassen sich die einzelnen Fehlerarten nicht unterscheiden.

Aufgabe der Erfindung ist es, eine Vorrichtung zur Kontrolle eines laufenden Einzelfadens zur Verfügung zu stellen, welche Unregelmässigkeiten differenziert nach Fehlerarten erfasst, insbesondere sogenannte Flusen, oder abstehende Fibrillen und/oder Dünnstellen oder Dickstellen an Monofilamenten.

Die Aufgabe wird erfindungsgemäss dadurch gelöst, dass der erste Messkopf mit einer Aussparung und der zweite Messkopf mit einer Aussparung versehen sind, wobei das Querschnittprofil der Aussparung des zweiten Messkopfes grösser als die Aussparung des ersten Messkopfes ist.

Am ersten Messkopf kann die Empfindlichkeit variabel eingestellt werden. Da der Faden nur knapp in der Aussparung versenkt ist, werden mit dem ersten Kopf mit tief eingestellter Empfindlichkeit nur grössere Garnunregelmässigkeiten, z.B. Flusen gezielt erfasst. Der zweite Messkopf erfasst bereits alle Ereignissse, d.h., auch gebrochene Fibrillen, welche die obere Kante der Aussparung überragen. Der Faden ist, je nach gewünschtem Erfassungsgrad, in der Aussparung des zweiten Messkopfes versenkt, so dass gleichzeitig in einem Durchgang verschiedene Arten von Fehlern und Unregelmässigkeiten am Faden feststellbar sind.

Kein bekanntes Verfahren ist in der Lage ausschliesslich nur den Schattenwurf des vom laufenden Faden abstehenden Fehlers zur Signalauswertung und somit auch zur Fehlerdifferenzierung zu verwerten.

Das automatische Unterscheiden der verschiedenen Fehler ermöglicht eine Klassierung und bringt damit Vorteile inbezug auf die Qualitätssicherung eines Garnes. Die Folgen der automatischen Kontrolle sind Personaleinsparungen bei der Überwachung und Begutachtung von Fehlern, sowie eine sofortige Aussage über die aktuelle Qualität der laufenden Produktion.

Es sind verschiedene Querschnittprofile, wie z.B. solche mit V-förmigem oder rechtwinkligem Querschnitt zur Durchführung der Messungen geeignet. Es hat sich als besonders vorteilhaft erwiesen, wenn das Querschnittsprofil des zweiten Messkopfes wenigstens 1.02 mal, insbesondere 2 -5 mal, bevorzugt 1.5 - 3 mal tiefer ist als das Querschnittsprofil des ersten Messkopfes. Die Nutbreite soll mindestens so gross wie der Fadenquerschnitt, insbesondere 1.1 - 10 mal, bevorzugt 1.5 - 2 mal grösser sein. Mit der erfindungsgemässen Vorrichtung können auf einfache Weise auch Dickstellen an Monofilamenten bestimmt und klassiert werden.

Es ist zweckmässig, die Aussparungen als austauschbare Einschübe auszubilden. Damit wird ermöglicht, mit einer Grundeinheit, kombiniert mit den verschiedenen Einschüben ein ganzes Spektrum von Titern abzudecken. In der Praxis sind Titer im Bereich von 0,5 bis 10′000 dtex, bevorzugt, 5 bis 3′000 dtex mit der Vorrichtung der Erfindung überwachbar.

Die Erfindung soll anhand einer Zeichnung näher beschrieben werden. Es zeigen:
- Fig. 1: eine schematische Anordnung der erfindungsgemässen Überwachungs- und Messvorrichtung
- Fig. 2a: einen Schnitt durch den ersten Messkopf im Bereich des Fadenlaufes
- Fig. 2b: einen Schnitt durch den zweiten Messkopf im Bereich des Fadenlaufes
- Fig. 3: a eine Fluse (Garnfehler, Verdickung)
b eine gebrochene Fibrille (Einzelfaser)
c eine abstehende Fibrille (Schlaufe)

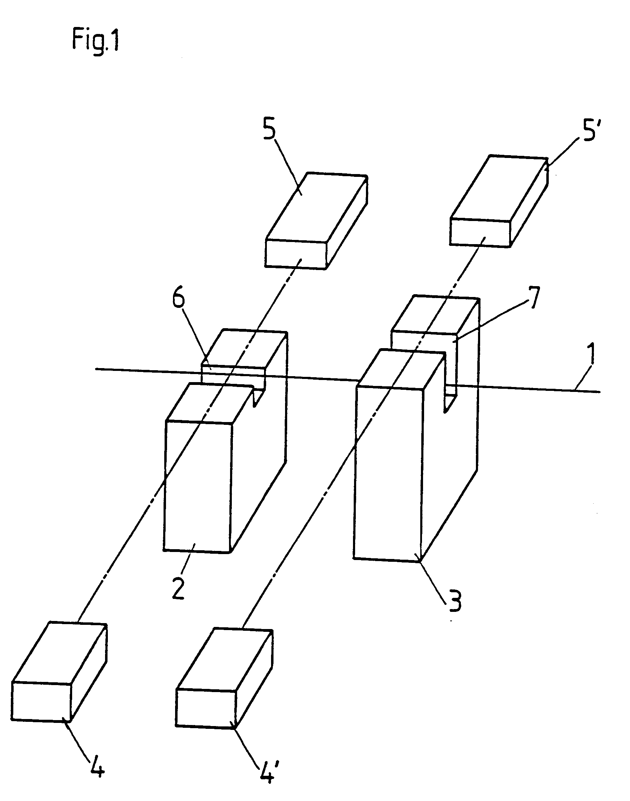

In Figur 1 ist mit den Bezugszeichen 1 ein laufender Faden bezeichnet. Der Faden kann ein Multifilament, Monofilament oder ein Fasergarn sein. Einem ersten Messkopf 2 sind ein Strahlensender 4 und ein Strahlenempfänger 5; einem zweiten Messkopf 3 sind ein Strahlensender 4′ und ein Strahlenempfänger 5′ zugeordnet. Der Messkopf 2 ist mit einer Aussparung 6, und der Messkopf 3 ist mit einer Aussparung 7 versehen. Die Ausparung 7 kann in Form eines Exzenters oder eines Keils oder einer sonst einstellbaren Einrichtung vorgesehen sein.

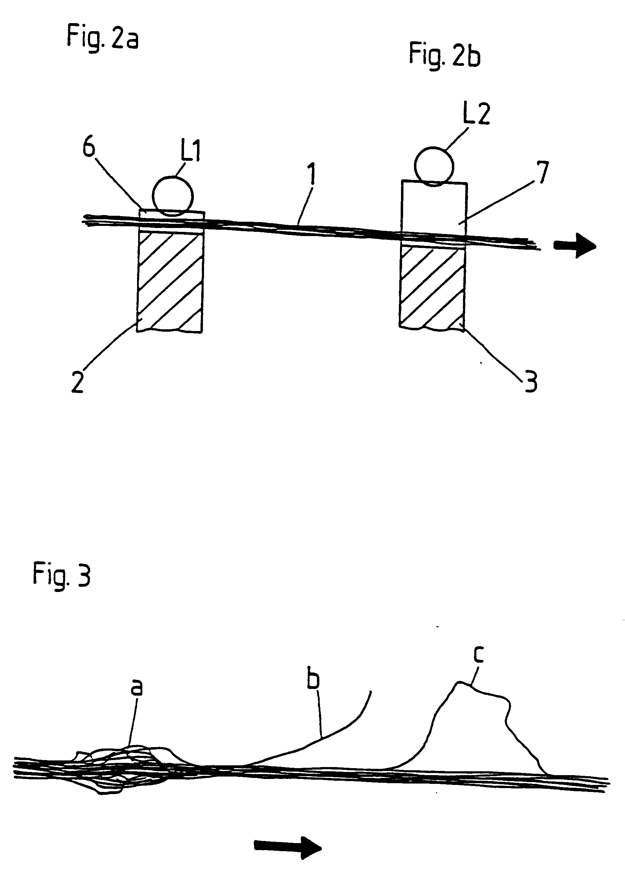

In Fig. 2a ist mit L1 ein Lichtstrahl vom Strahlensender 4 bezeichnet, welcher auf den Messkopf 2 gerichtet ist. Die geringe Tiefe der Aussparung 6 lässt erkennen, dass ein Faden 1 nur knapp darin versenkt ist. Hier werden nur grössere Garnfehler z.B. Flusen (Fig. 3a) jedoch keine Einzelfibrillen (Fig. 3b) erfasst.

In Fig. 2b ist mit L2 ein Lichtstrahl vom Strahlensender 4′ bezeichnet, welcher auf den Messkopf 3 gerichtet ist. Hier ist der Faden 1 tief in der Aussparung 7 des Messkopfes 3 versenkt. Es werden alle Ereignisse ab Einzelfibrille erfasst, welche die Kante A überragen.

Die Tiefe der Versenkung des Fadens 1 kann je nach gewünschtem Erfassungsgrad erfolgen.

In Fig. 3 sind mit a) Flusen, mit b) abstehend gebrochene Fibrillen und mit c) abstehende Schlaufen bezeichnet. Der Vorteil der Erfindung besteht in der genauen Erfassung und automatischen Registrierung von Unregelmässigkeiten an einem laufenden Faden, wobei die Einzelereignisse auch nach ihrer Art erfasst werden. Die Erfindung ist besonders zur Qualitätüberwachungsprüfung eines Einzelfadens geeignet.

Anstelle von zwei hintereinandergeschalteten Messköpfen können zum Abstufen der Klassierungen mehrere Messköpfe mit variabler Empfindlichkeit hintereinander angeordnet sein. Die erfindungsgemässe Überwachungseinrichtung kann auch mit einem integrierten Fadenbruchwächter kombiniert werden. Sie kann besonders vorteilhaft in einem geerdeten, geschlossenen, kompakten Gehäuse untergebracht sein. Falschmeldungen, die beispielsweise durch unruhigen Fadenlauf hervorgerufen werden, können im Bedarfsfall mittels einer Betägungsschaltung wesentlich vermindert werden.

## Patentansprüche

1. Vorrichtung zur photoelektrischen Überwachung eines laufenden Fadens (1) auf Unregelmässigkeiten, bestehend aus in Fadenlaufrichtung hintereinander angeordneten Messköpfen, wobei einem ersten Messkopf (2) ein Strahlensender (4) und ein Strahlenempfänger (5) und einem zweiten Messkopf (3) ein Strahlensender (4′) und ein Strahlenempfänger (5′) zugeordnet sind, dadurch gekennzeichnet, dass der erste Messkopf (2) mit einer Aussparung (6) und der zweite Messkopf (3) mit einer Aussparung (7) versehen sind, wobei das Querschnittprofil der Aussparung (7) des zweiten Messkopfes (3) grösser als die Aussparung (6) des ersten Messkopfes (2) ist.

2. Vorrichtung nach Anspruch 1, dadurch gekennzeichnet, dass das Querschnittprofil der Aussparung (7) wenigstens 2,0 % grösser ist als das Querschnittprofil der Aussparung (6).

3. Vorrichtung nach Anspruch 1, dadurch gekennzeichnet, dass das Querschnittprofil V-förmig ausgebildet ist.

4. Vorrichtung nach Anspruch 1, dadurch gekennzeichnet, dass das Querschnittprofil rechtwinklig ausgebildet ist.

5. Vorrichtung nach Anspruch 1, dadurch gekennzeichnet, dass die Nutbreite 1.1 bis 10 mal grösser als der Querschnitt des Fadens (1) ist.

6. Vorrichtung nach Anspruch 1, dadurch gekennzeichnet, dass die Aussparungen (6,7) als austauschbare Einschübe ausgebildet sind.

## Claims

1. A device for photo-electrically monitoring a moving yarn (1) for irregularities, consisting of sensor heads connected in series in the direction of yarn movement, in which a beam transmitter (4) and a beam receiver (5) are aligned with a first sensor head (2) and a beam transmitter (4′) and a beam receiver (5′) are aligned with a second sensor head (3), thereby characterized in that the first sensor head (2) is equipped with a recess (6) and the second sensor head is equipped with a recess (7) where the cross-sectional depth of the recess (7) of the second sensor head (3) is greater than the recess (6) of the first sensor head (2).

2. A device according to claim 1, thereby characterized in that the cross-sectional depth of the recess (7) is at least 2.0 % greater than the cross-sectional depth of the recess (6).

3. A device according to claim 1, wherein the cross sectional profile of the recess is V-shaped.

4. A device according to claim 1, where the cross sectional profile of the recesses are right-angled.

5. A device according to claim 1 wherein the recess (6) through the first sensor head is 1.1 to 10 times wider than the cross section of the yarn.

6. A device according to claim 1, thereby characterized in that the recess (6,7) is interchangeable optional units.

## Revendications

1. Dispositif pour le contrôle photoélectrique d'irrégularités d'un fil défilant (1) comprenant des têtes de mesures lesquelles sont disposées l'un après l'autre dans le sens de marche du fil, où pour une première tête de mesure (2) est attribuée un émetteur (4) et un récepteur (5) et pour la deuxième tête de mesure (3) est attribuée un émetteur (4′) et un récepteur (5′), caractérisé en ce que la première tête de mesure (2) comprend un évidement (6) et la deuxième tête de mesure (3) un évidement (7) où la section transversale de l'évidement (7) de la deuxième tête de mesure (3) et plus grande que l'évidement (6) de la première tête de mesure (2).

2. Dispositif selon la revendication 1, caractérisé en ce que la section transversale de l'évidement (7) est au moins 2 % supérieure à la section transversale de l'évidement (6).

3. Dispositif selon la revendication 1, caractérisé en ce que la section transversale est en forme de V.

4. Dispositif selon la revendication 1, caractérisé en ce que la section transversale forme un angle droit.

5. Dispositif selon la revendication 1, caractérisé en ce que la largeur de la rainure est de 1,1 à 10 fois supérieure au diamètre du fil (1).

6. Dispositif selon la revendication 1, caractérisé en ce que les évidements (6,7) sont des dispositifs pouvant être remplacés.
